# EUROPEAN PATENT APPLICATION

(11) **EP 4 699 515 A1**
(43) Date of publication of application: **25.02.2026**
(21) Application number: 25197671.8
(22) Date of filing: 22.08.2025
(51) Int. Cl.: A61B 1/00, G06F 1/3203, G06F 1/3206, G06F 1/3234, G06T 7/00, G06T 7/10, G06T 7/12, G06V 10/25

(54) **SYSTEMS AND METHODS FOR DISPLAYING IMAGES COMPRISING STATIC CONTENT BY A MEDICAL DISPLAY DEVICE IN A MEDICAL ENVIRONMENT**

(30) Priority: 23.08.2024 US 202463686657 P
(71) Applicant: Stryker Corporation, Portage, MI 49002 (US)
(72) Inventor: MISTRY, Neel, Portage, 49002 (US); TEA, David, Portage, 49002 (US); STEIN, Richard, Portage, 49002 (US)
(74) Representative: V.O.

(57) **Abstract**

Disclosed are systems and methods for avoiding/reducing prolonged exposure of a medical display device to static content by determining whether an image to be displayed by the medical display device comprises static content and operating the medical display device in static imaging mode accordingly. The method may comprise receiving an image for display by the medical display device and generating an image representation of (at least) a region of interest of the received image. The image representation may be compared to a pre-determined image representation and determined that the image representation corresponds to the pre-determined image representation. Since the image representation corresponds to the pre-determined image representation, the image comprises static content, and the medical display device is operated in a static imaging mode. In static imaging mode, the medical display device may display a message, operate in a low power mode, and/or perform a pixel refresh.

## Description

### CROSS-REFERENCE TO RELATED APPLICATIONS

This application claims the benefit of U.S. Provisional Application No. 63/686,657, filed August 23, 2024, the entire contents of which are hereby incorporated by reference herein.

### FIELD

The present invention relates to operating a medical display device, particularly for increasing the lifetime of the medical display device, in a medical environment.

### BACKGROUND

Surgery generally involves the use of one or more multimedia source devices, such as a medical display device that allows a medical operator (*e.g*., surgeon) to visualize the internal area of a subject's body being viewed by a camera (such as a high-definition (HD) camera coupled to an endoscope inserted into the subject, or a fluorescent camera used for open surgery type tasks). The medical display device helps provide the medical operator with a clear and precise view within the subject's body. In some instances, the signal output from the camera is transmitted to and displayed on the medical display device as an image.

The medical display device may be a light emitting diode (LED) display, an organic LED (OLED) display, or a liquid crystal display (LCD), for example. In some instances, the image displayed by the medical display device comprises static content. For example, the medical display device may display an image indicating the status of the system configuration, such as when one or more devices are disconnected (*e.g*., the camera is disconnected from an image and video capture device or a device console) or are powered off (*e.g.*, the camera is not turned on). The image may comprise static content. When a medical display device is exposed to prolonged static content, the medical display device may experience issues such as image retention and permanent burn in. Shorter term exposure can be corrected through built-in display functionality, but long-term exposure may not be correctable and may lead to permanent image burn in. Avoiding or reducing prolonged exposure of the medical display device to static content can be beneficial to increasing the lifetime of the medical display device.

### SUMMARY

According to various aspects, systems and methods include avoiding or reducing prolonged exposure of a medical display device to static content. The prolonged exposure of a medical display device to static content may be avoided or reduced by determining whether an image to be displayed by the medical display device comprises static content and operating the medical display device in static imaging mode accordingly. When operating in static imaging mode, the medical display device may display messages (or use an indicator such as an LED indicator), operate in low power mode (*e.g.*, cause a reduced brightness of the medical display device), and/or perform a pixel refresh. Operating the medical display device in a low power mode or performing a pixel refresh helps reduce or avoid exposure to static content.

According to some examples, a method for operating a medical display device comprises: receiving an image for display by the medical display device; generating an image representation of at least a region of interest of the received image; comparing the image representation to a pre-determined image representation; determining whether the image representation corresponds to the pre-determined image representation; and if it is determined that the image representation corresponds to the pre-determined image representation operating the medical display device in a static imaging mode.

In any of the examples, the region of interest of the received image may be a subset of the received image.

In any of the examples, the region of interest of the received image can comprise one or more of: a color bar, a status depiction, and a logo.

In any of the examples, the region of interest of the received image can comprise one or more of: a depiction of a connector, a depiction of a Wi-Fi symbol, a depiction of a multimedia cable, and a depiction of a medical device.

In any of the examples, the method can further comprise dynamically configuring parameters of the region of interest of the received image.

In any of the examples, the image can comprise the region of interest and an other region, wherein the other region comprises dynamic content.

In any of the examples, the method can further comprise generating the pre-determined image representation by averaging a plurality of pre-determined image representations having a plurality of resolutions.

In any of the examples, generating the image representation can comprise determining luminance values for the region of interest of the received image.

In any of the examples, the pre-determined image representation can correspond to a third-party pre-determined image representation.

In any of the examples, the image representation can comprise an image histogram and the pre-determined image representation comprises a pre-determined image histogram.

In any of the examples, the region of interest of the received image can comprise static content when: an image and video capture device is decoupled from a device console; or a device console is decoupled from a medical device.

In any of the examples, being decoupled from a medical device comprises being disconnected from the medical device, or the medical device being off or in a low power mode.

In any of the examples, the pre-determined image representation can be stored in a memory of the medical display device.

In any of the examples, operating the medical display device in the static imaging mode can comprise one or more of: causing the medical display device to display a message; operating the medical display device in a low power mode; or performing a pixel refresh with the medical display device powered off.

In any of the examples, the method can further comprise waiting a timeout period after determining that the image representation corresponds to the pre-determined image representation before operating the medical display device in the static imaging mode.

In any of the examples, the method can further comprise receiving a bypass signal; and foregoing the comparing step and the determining step.

According to some examples, a system for displaying a medical image or video comprises: a medical display device configured to: receive an image for display; generate an image representation of at least a region of interest of the received image; compare the image representation to a pre-determined image representation; determine whether the image representation corresponds to the pre-determined image representation; and if it is determined that the image representation corresponds to the pre-determined image representation operate in a static imaging mode.

In any of the examples, the image can comprise the region of interest and an other region, and the region of interest can be a subset of the received image, and the other region can comprise dynamic content.

In any of the examples, the system can further comprise an image and video capture device; a device console; and a medical device, wherein the region of interest of the received image comprises static content when the image and video capture device is decoupled from the device console, or the device console is decoupled from the medical device.

According to some examples, a computer program product includes computer-implementable instructions configured to be executed by one or more processors of a system, wherein executing the instructions causes the system to: receive an image for display by the medical display device; generate an image representation of at least a region of interest of the received image; compare the image representation to a pre-determined image representation; determine whether the image representation corresponds to the pre-determined image representation; and if it is determined that the image representation corresponds to the pre-determined image representation operate the medical display device in a static imaging mode. According to some examples, a non-transitory computer-readable storage medium stores such a computer program product.

It will be appreciated that any of the variations, aspects, features, and options described in view of the system apply equally to the method and computer program product, and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1 illustrates an example operating room, according to some aspects.
FIG. 2 illustrates an example system comprising an image and video capture device, input devices, and output devices, according to some aspects.
FIGs. 3A-3D illustrate example system configurations having a decoupled device console and/or medical device, according to some aspects.
FIG. 4 illustrates an example image representation-based method, according to some aspects.
FIG. 5 illustrates an example of different modes of a medical display device, according to some aspects.
FIG. 6A illustrates an example medical display device configured to display an image comprising static content, according to some aspects.
FIG. 6B illustrates an example medical display device configured to display overlays, according to some aspects.
FIG. 7 illustrates an example table showing an image histogram of an image to be displayed by the medical display device, according to some aspects.
FIG. 8 illustrates an example table showing an image histogram of an image to be displayed by the medical display device, according to some aspects.
FIG. 9 illustrates an example computing system used for performing any of the methods and systems described herein, according to some aspects.

### DETAILED DESCRIPTION

Reference will now be made in detail to implementations and various aspects and variations of systems and methods described herein. Although several example variations of the systems and methods are described herein, other variations of the systems and methods may include aspects of the systems and methods described herein combined in any suitable manner having combinations of all or some of the aspects described.

Systems and methods according to the principles described herein can avoid or reduce prolonged exposure of a medical display device to static content. The prolonged exposure of a medical display device to static content may be avoided or reduced by operating the medical display device in a static imaging mode when the image for display by the medical display device comprises static content. In some aspects, the medical display device may determine whether the image comprises static content by generating an image representation of at least a region of interest of the received image and comparing the image representation to a pre-determined image representation. The image representation (of the received image) may correspond to (*e.g.*, have values that match, or are within a threshold deviation from) the pre-determined image representation. When operating in static imaging mode, the medical display device may display messages (or use an indicator such as an LED indicator), operate in a low power mode (*e.g.*, cause a reduced brightness of the medical display device), and/or perform a pixel refresh, thereby helping to reduce or avoid exposure of the medical display device to static content.

In some instances, the medical display device may receive a "no signal" image comprising static content. The "no signal" image may be generated when one or more devices of the disclosed system are decoupled (disconnected from, or the medical device is off or in a low power mode). The "no signal" image may comprise a status depiction indicative of a disconnection, or a medical device being off or in a low power mode. The system may comprise a plurality of devices that should be connected including, but not limited to, a medical display device, an image and video capture device, a device console, and a medical device (*e.g.*, a camera or another medical device). In some examples, the medical device may be decoupled from the image and video capture device or the device console, and the medical display device may receive an image comprising a depiction of a connector or a depiction of the medical device to indicate that the medical device is disconnected from the image and video capture device or the device console, or that the medical device is off (or in low power mode). In some aspects, the device console may be decoupled from the image and video capture device, and the medical display device may receive an image comprising a depiction of a connector or a depiction of a device console to indicate the decoupling. In some aspects, the medical display device may be decoupled from any device, such as an image and video capture device, a device console, or a Wi-Fi router. The medical display device may receive an image having a region of interest comprising a depiction of a connector, a depiction of a Wi-Fi symbol, a depiction of a multimedia cable, or a depiction of a medical device (*e.g.*, a camera).

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or medical display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability. Suitable processors include central processing units (CPUs), graphical processing units (GPUs), field-programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

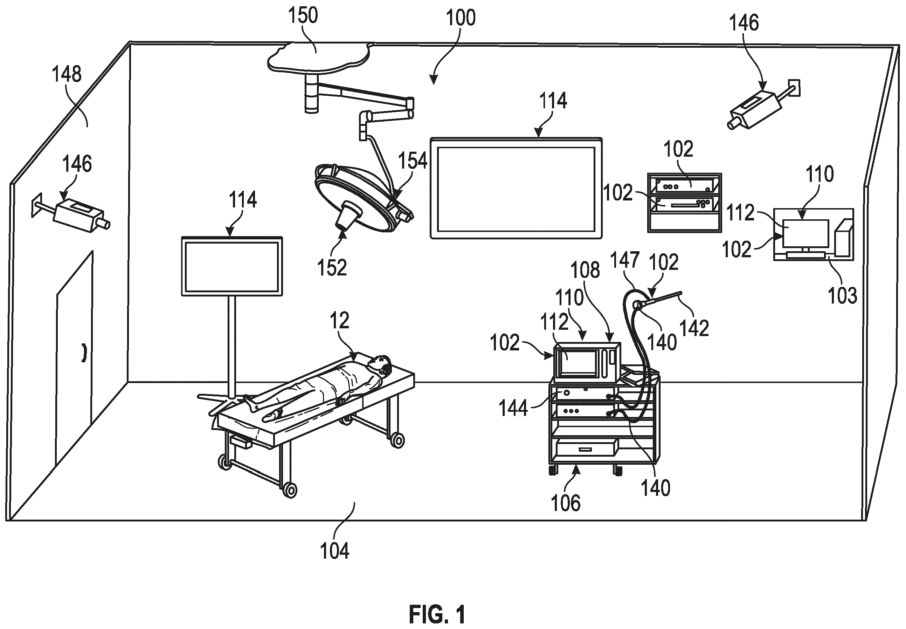

FIG. 1 illustrates an example operating room in a medical environment, according to some aspects. The operating room 100 comprises at least one medical device 102 to assist in performing a medical or surgical procedure and/or for recording keeping purposes. For example, the medical device 102 may be used to input or receive (*e.g*., from electronic medical records (EMRs), from electronic health records (EHRs), communicated in real-time from another system, etc.) patient information for use with information or images saved onto the medical device 102, displaying information or images from the medical device 102, sending to other medical devices 102, or a combination thereof. In some aspects, the medical device 102 may be used to record patient information, including storing the information or images in an EMR, EHR, or other type of file.

The medical device 102 located within the operating room 100 can include any device that is capable of saving information related to a subject 12. The medical device 102 may or may not be coupled to a network that includes records of the subject 12. The medical device 102 may include a computer system 110 (*e.g.*, a desktop computer, a laptop computer, a tablet device, etc.) having an application server. The computer system 110 can have a motherboard that includes one or more processors or other similar control devices, as well as one or more memory devices. The processor controls the overall operation of the computer system 110 and can include hardwired circuitry, programmable circuitry that executes software, or a combination thereof. The processor may, for example, execute software stored in the memory device. The processor may include, for example, one or more general- or special-purpose programmable microprocessors and/or microcontrollers, application specific integrated circuits (ASICs), programmable logic devices (PLDs), programmable gate arrays (PGAs), or the like. The memory device may include any combination of one or more random access memories (RAMs), read-only memories (ROMs) (which may be programmable), flash memory, and/or other similar storage devices. Patient information may be inputted into the computer system 110 for use with the computer system 110 (*e.g.*, for making an operative note during the medical or surgical procedure on the subject 12 in the operating room 100) and/or the computer system 110 can transmit the patient information to another medical device 102 (via either a wired connection or wirelessly).

The medical device 102 can be positioned in the operating room 100 on a table (stationary or portable), a floor 104, a portable cart 106, an equipment boom, and/or shelving 103. FIG. 1 illustrates two computer systems 110: a first computer system 110 in the form of a desktop computer shelving 103, and a second computer system 110 incorporated into an image and video capture device 108 on a portable cart 106. It is contemplated that the computer system 110 can be on the portable cart 106 (*e.g.*, on the same cart 106 as an image and video capture device 108 or on a separate cart). In some aspects, the image and video capture device 108 and/or associated router(s) (not shown) may be located in a room outside of the operating room 100, such as in a closet. In some other aspects, the image and video capture device 108 and/or associated router(s) (not shown) may be located in a cabinet inside the operating room 100. Further, examples of the disclosure may include any number of computer systems.

The image and video capture device 108 may be capable of recording images, recording videos, displaying images, displaying videos, recording audio, outputting audio, or a combination thereof. In some aspects, patient information can be input into the image and video capture device 108 for adding to the images and videos recorded and/or displayed by the image and video capture device 108. The image and video capture device 108 can include internal storage (*e.g.*, a hard drive, a solid-state drive, etc.) for storing the captured images and videos. The image and video capture device 108 can also display any captured or saved images (*e.g*., from the internal hard drive) or on an associated touchscreen medical display device 112 and/or an additional medical display device 114 coupled to the image and video capture device 108 via either a wired connection or wirelessly. It is contemplated that the image and video capture device 108 could obtain or create images of the subject 12 during a medical or surgical procedure from a variety of sources (*e.g*., from video cameras, video cassette recorders, X-ray scanners (which convert X-ray films to digital files), digital X-ray acquisition apparatus, fluoroscopes, computed tomography (CT) scanners, magnetic resonance imaging (MRI) scanners, ultrasound scanners, charge-coupled (CCD) devices, and other types of scanners (handheld or otherwise)). If coupled to a network, the image and video capture device 108 can also communicate with a picture archiving and communication system (PACS), as is well known to those skilled in the art, to save images and video in the PACS and for retrieving images and videos from the PACS. The image and video capture device 108 can couple and/or integrate with, *e.g.*, an electronic medical records database and/or a media asset management database.

The image and video capture device 108 is capable of displaying images and videos on a touchscreen medical display device 112 and/or on an additional medical display device 114 captured live by cameras (*e.g*., a video camera 140 coupled to an associated endoscope 142, which communicates with a camera control unit (CCU) 144 via a fiber optic cable 147, with the camera control unit 144 communicating via wires or wirelessly with the image and video capture device 108) and/or replayed from recorded images and videos. It is further contemplated that the image and video capture device 108 can display images and videos on the touchscreen medical display device 112 and/or on the additional medical display device 114 captured live by a room camera 146 fixed to walls 148 or a ceiling 150 of the operating room 100 (*e.g.*, a room camera 146 as shown, or a camera 152 in an overhead light 154). In some aspects, the image and video capture device 108 can be used to integrate, annotate, and/or correct images.

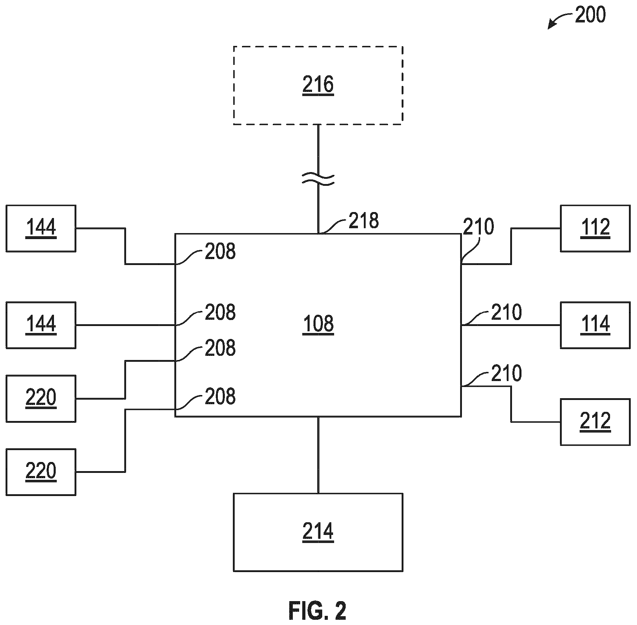

FIG. 2 illustrates a block diagram of an example system 200 comprising an image and video capture device, input devices, and output devices, according to some aspects. The image and video capture device 108 receives one or more multimedia signals from one or more CCUs 144 to be routed to one or more medical display devices 112 or 114. The one or more CCUs 144 may generate image data associated with treatment of a patient (*e.g*., subject 12 shown in FIG. 1). The image data can be images or videos generated during treatment of the patient in support of one or more medical procedures, such as video captured by an endoscopic camera. Examples of source devices include, without limitation, endoscopic systems, open field imaging systems, x-ray systems such as intraoperative c-arm systems, computer tomography (CT) systems, ultrasound systems, magnetic resonance imaging (MRI) systems, and nuclear medicine systems.

In some aspects, the image and video capture device 108 may receive data from one or more non-imaging devices 220 that may be used in connection with (*e.g*., during) a medical imaging session (*e.g*., surgical procedure) and may provide information that may be relevant for display during a medical imaging session. Non-limiting examples of non-imaging devices include insufflators, illumination controllers, and voice control systems.

The image and video capture device 108 may receive a multimedia signal from the one or more CCUs 144 through one or more input ports 208. The image and video capture device 108 generates one or more display feeds using the received multimedia signal and transmits the one or more display feeds to one or more medical display devices 112 or 114 via one or more output ports 210. For example, the image and video capture device 108 may generate a display feed that includes enhanced imaging of tissue of a patient based on imaging generated by one or more CCUs 144, and the enhanced imaging may be displayed on one or more medical display devices 112 or 114 to assist a practitioner during treatment of the patient. In some aspects, the image and video capture device 108 can operate with a router to route multimedia signals from the input port(s) 208 to the output port(s) 210. The format of the multimedia data may include analog, digital, HD format, UHD format (*e.g.*, 4K or 8K video), or the like. In some aspects, the image and video capture device 108 may be configured to control a signal router or a network router that converts the multimedia signal to an IP multimedia stream (*e.g*., using encoders and decoders). In some aspects, the signal router may be capable of routing multimedia signals without use of a network router and does not require conversion to an IP multimedia stream. The image and video capture device 108, signal router, or both may operate with (*e.g*., integrated with or located externally) a circuit that switches between multimedia inputs for a given multimedia output.

Image and video capture device 108 may also transmit display feeds to one or more recording devices 212 for recording enhanced imaging for later retrieval. Input ports 208 and output ports 210 may be any suitable type of data transmission ports, such as digital visual interface (DVI) ports, high-definition multimedia interface (HDMI) ports, DisplayPort (DP) ports, VGA ports, RS232 ports, IP (network, *e.g*., Ethernet) ports, and the like.

Image and video capture device 108 may be coupled to one or more networks 216 via one or more network connections 218. The one or more networks 216 may include a local network such as a hospital information system, or a wider network such as a wide area network or the internet. A network connection 218 can be a wired connection, such as an Ethernet connection, or a wireless network connection, such as a Wi-Fi connection. In some aspects, the image and video capture device 108 may access the one or more networks 216 to retrieve configuration data stored at a network location for configuring the image and video capture device 108 for an imaging session, and/or may access the one or more networks to receive updated software and/or updated hardware files for processing imaging data. In some aspects, the image and video capture device 108 may access a database comprising information, such as EMRs, EHRs, or other patient data, for retrieval and/or storage.

One or more user interfaces 214 may be in communication with (*e.g*., connected to) the image and video capture device 108 for a user to provide input to the image and video capture device 108. The user may input data related to configuring the image and video capture device 108 for an imaging session. User input can include, for example, selection of a practitioner profile associated with an upcoming imaging session, selection of the type of imaging session or types of procedure to be performed during an imaging session, selection of which inputs (*e.g.*, multimedia inputs) are routed to which outputs (*e.g.*, multimedia outputs), predetermined routing selections (*e.g*., surgeon- or patient-specific, preset configurations), or any other relevant information such as the operation mode and associated parameters for the medical display device 114 (discussed in more detail below). The one or more user interfaces 214 may include a tablet, a keyboard, a mouse, a voice control system, a keypad, a touchscreen, or any combination thereof. In some aspects, the one or more user interfaces 214 may include one or more indicators, such as a signal input indicator (*e.g*., phase-locked loop (PLL) indicator) that indicates when an input port 208 comprises a multimedia signal.

In some aspects, the image and video capture device 108 processes received medical imaging data and any other relevant data and generates enhanced display feeds for display on one or more medical display devices 112 during an imaging session. According to some aspects, the image and video capture device 108 may combine multiple imaging sources into a single display feed, process received imaging data to generate richer imaging data, modify imaging data for better utilization of display space, and/or reconfigure the processing of imaging data depending on the needs and preferences of users from imaging session to imaging session.

Aspects of the disclosure include one or more systems and methods for enhancing the lifetime and integrity of one or more medical display devices. As a non-limiting example, in some aspects, a medical display device may be exposed to image(s) comprising static content. Displaying static content may lead to issues with the medical display device, such as image retention and burn in. The damage to the medical display device may be permanent, such as permanent damage due to exposure to prolonged static content. In some examples, image retention and permanent burn in may be due to exposing a medical display device (*e.g.*, an LED medical display device, an OLED medical display device, or an LCD medical display device) to prolonged static content. In some examples, the medical display device may display a still image indicative of a status of the system. For example, the medical display device 112 may receive a "no signal" (still) image indicative that one or more devices in the system are decoupled (such as disconnected or powered off or in a low power mode). The "no signal" image may comprise one or more of: a depiction of a connector, a depiction of a Wi-Fi symbol, a depiction of a multimedia cable, and a depiction of a medical device, shown in a region of interest of the image. In some examples, the region of interest may be a subset of the image received by the medical display device 112 (as discussed in more detail below). In some examples, a medical operator (*e.g.*, a surgeon) may use the medical display device to view a still image (*e.g.*, an x-ray image) comprising static content.

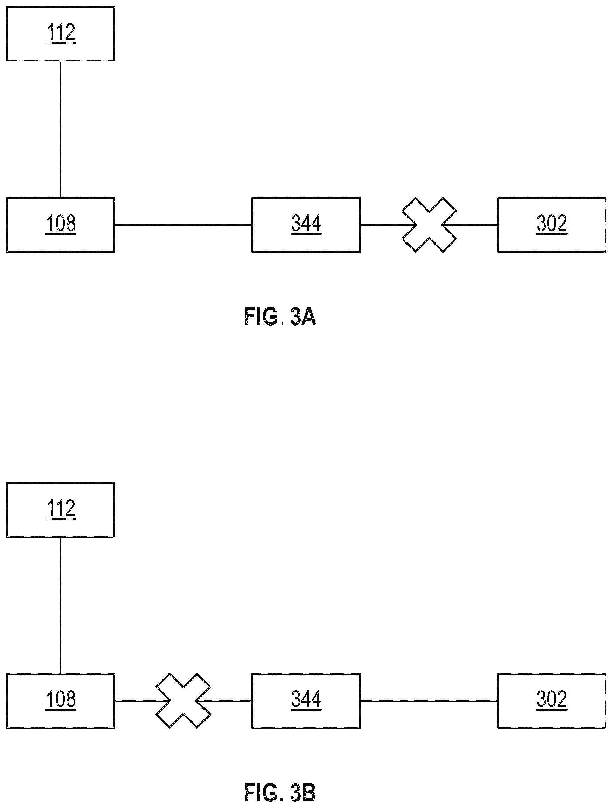

FIGs. 3A-3D illustrate non-limiting example system configurations having a decoupled device console and/or medical device, according to some aspects. As shown in FIG. 3A, the medical display device 112 may be connected to an image and video capture device 108. The image and video capture device 108 may be connected to a device console 344 (*e.g.*, a CCU 144), but the device console 344 may be decoupled from a medical device 302. The medical display device 112 may receive an image comprising static content (*e.g.*, a depiction of a connector, a depiction of a medical device, etc.) to indicate that the device console 344 is decoupled from the medical device 302 (*e.g.*, a camera 140 or another medical device 102). FIG. 3B illustrates an example system configuration where the medical display device 112 is connected to an image and video capture device 108, but the image and video capture device 108 is decoupled from the device console 344. The medical display device 112 may receive an image comprising static content (*e.g.*, a depiction of a connector or a depiction of a device console) to indicate that the image and video capture device 108 is decoupled from the device console 344.

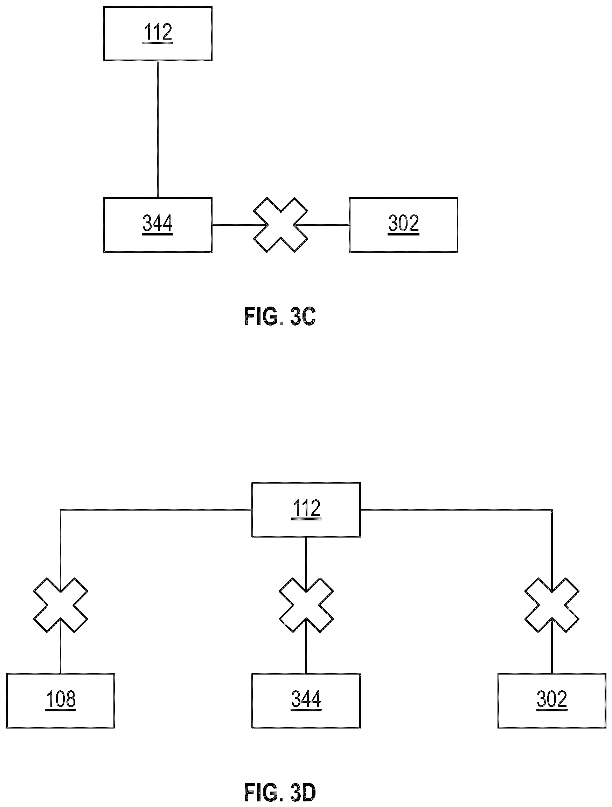

In some aspects, as shown in FIG. 3C, the medical display device 112 may be connected to the device console 344, but the device console 344 may be decoupled from a medical device 302. FIG. 3D illustrates another example system configuration where the medical display device 112 may be decoupled from any device (*e.g.*, an image and video capture device 108, a device console 344, a medical device 302, etc.). In some aspects, when a medical device is powered off or in a low power mode, the static content of the image received by the medical display device 112 may comprise a status depiction of a multimedia cable, for example.

The medical display device 112 may determine that the received image comprises static content by using, for example, an image representation-based method, a frame comparison-based method, or a bypass signal-based method, as described in more detail below. In response to the medical display device 112 receiving an image comprising static content, the medical display device 112 may operate in a static imaging mode. Although shorter term exposure of the medical display device 112 to static content can be corrected through built-in functionality (*e.g*., manually operating a pixel refresh) or varying the content in the video (comprising a plurality of images), in some aspects, manually performing pixel refresh may not be suitable enough to avoid permanent image burn in of the medical display device 112.

Examples of the disclosure may comprise one or more methods (and associated systems) for minimizing the exposure of the medical display device 112 to static content and/or prolonging the integrity of the medical display device 112. In some aspects, the medical display device 112 may employ an image representation-based method that generates an image representation (*e.g*., histogram) of a region of interest of the image received by the medical display device 112. The image representation may be compared to a pre-determined image representation, and it may be determined that the image representation corresponds to *(e.g.,* has values that match, or are within a threshold deviation of) the pre-determined image representation.

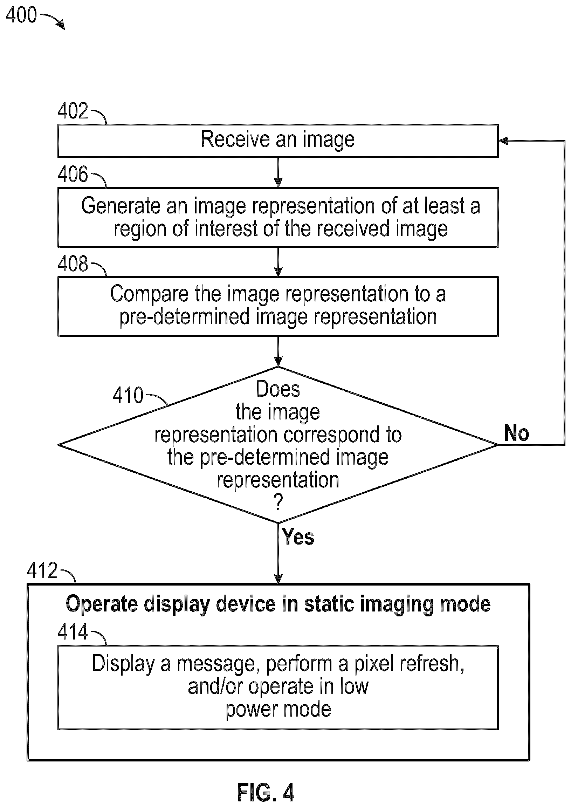

FIG. 4 illustrates an example image representation-based method, according to some aspects. The method 400 may comprise receiving an image at step 402 and generating an image representation of at least a region of interest of the received image at step 406. In some aspects, the region of interest may be a subset of the received image. The region of interest and the image representation are discussed in more detail below.

In step 408, the image representation may be compared to a pre-determined image representation. In some examples, the pre-determined image representation is representative of a "no signal" image. In some aspects, the pre-determined image representation may be stored in the memory of the medical display device 112, and the medical display device 112 may retrieve the pre-determined image representation for performing the comparison of step 408. In some aspects, the medical display device 112 may store a plurality of pre-determined image representations, and step 408 may comprise comparing the image representation to one or more pre-determined image representations. The plurality of pre-determined image representations may comprise different status depictions, for example. Examples of the disclosure include the medical operator providing user input, *e.g.*, a selection of a status depiction for the pre-determined image representation.

In some aspects, the pre-determined image representation may be generated by and/or stored in the memory of another device (*e.g.*, an image and video capture device 108, a device console 344, a medical device 102). For example, the pre-determined image representation may correspond to a third-party pre-determined image representation. A device (*e.g.*, an image and video capture device 108, a device console 344, a medical device 102) may transmit the pre-determined image representation to the medical display device 112. In some examples, the medical display device 112 may be configured to capture a pre-determined image comprising static content, generate a pre-determined image representation based on the captured pre-determined image, and store the pre-determined image representation in the memory of the medical display device 112. In some aspects, the medical operator may provide user input (*e.g.*, via a user interface presented to the medical operator on a display of the medical display device 112) that causes the medical display device 112 to capture the pre-determined image. In some aspects, another device (*e.g.*, a remote computing device via network 216) may cause the medical display device 112 to capture the pre-determined image.

In step 410, the method determines that the image representation corresponds to the pre-determined image representation. Step 410 may comprise comparing values of regions in the image representation to values of corresponding regions in the pre-determined image representation. In some aspects, the image representation corresponds to the pre-determined image representation when the values of the sub-regions/region of interest in the image representation match or are similar (within a threshold deviation) to the values of the corresponding sub-regions/region of interest in the pre-determined image representation. If the image representation corresponds to the pre-determined image representation, then the system determines that the medical display device 112 has received a "no signal" image comprising static content.

If the medical display device 112 received an image comprising static content, then the medical display device 112 may be configured to operate in a static imaging mode (step 412), where one or more steps are taken to minimize exposure of the medical display device 112 to static content. Operating the medical display device 112 in static imaging mode can comprise one or more of: causing the medical display device 112 to display a message (or use an indicator such as an LED indicator), operating the medical display device 112 in a low power mode, and performing a pixel refresh with the medical display device 112 powered off (step 414). In some examples, the method may include waiting a timeout period after determining that the image representation corresponds to the pre-determined image representation (step 410) before operating the medical display device 112 in static imaging mode (step 412). For example, the medical display device 112 may be operated in static imaging mode after a timeout period of five minutes. In this manner, the medical operator may be given a chance to see a status depiction in the region of interest of the image that indicates there is a disconnection or a medical device that is off. In some aspects, during the timeout period, the medical display device 112 may continuously determine that the received images comprise static content. After the timeout period, the medical display device 112 can display a message (or uses an indicator such as an LED indicator) informing the medical operator that static content has been detected. The medical display device 112 may then wait a static imaging mode period after displaying the message (or using the indicator) before operating the medical display device 112 in a low power mode or performing a pixel refresh. For example, the medical display device 112 may be operated in a low power mode or perform a pixel refresh after a static imaging mode period of 120 seconds. In other aspects, the timeout period and/or static image mode period may be user configurable (*e.g*., by a medical operator, system administrator, a third-party, etc.). In other aspects, the timeout period and/or static imaging mode period may span any length of time suitable for notifying a medical operator that there is a decoupling in the system and/or waiting to operate the medical display device 112 in a low power mode or performing a pixel refresh, respectively.

In some aspects, during the timeout period, the method may include determining that an image received by the medical display device 112 comprises dynamic content (e.g. an image received during the timeout period does not comprise static content). The method may return to step 402, and the medical display device 112 may then operate in imaging mode. In the imaging mode, the medical display device 112 may display the incoming image/video.

In static imaging mode, the medical display device 112 may periodically (*e.g*., every video frame) determine whether the medical display device 112 should remain in static imaging mode. In some aspects, the medical display device 112 may determine whether it should remain in static imaging mode by generating an image representation of an image (*e.g.*, step 406) and comparing the image representation to a pre-determined image representation (*e.g*., step 408). Examples of the disclosure include performing one or more steps of method 400 automatically (*e.g*., without requiring user input).

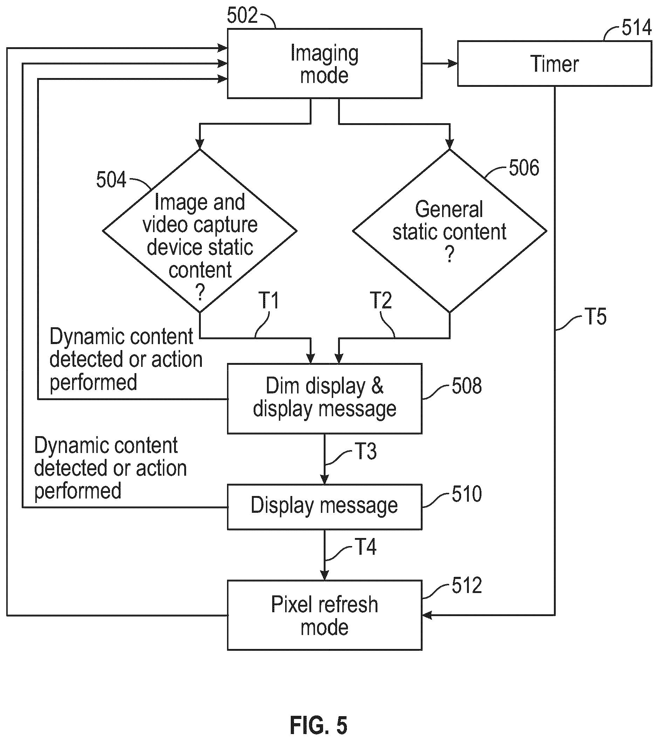

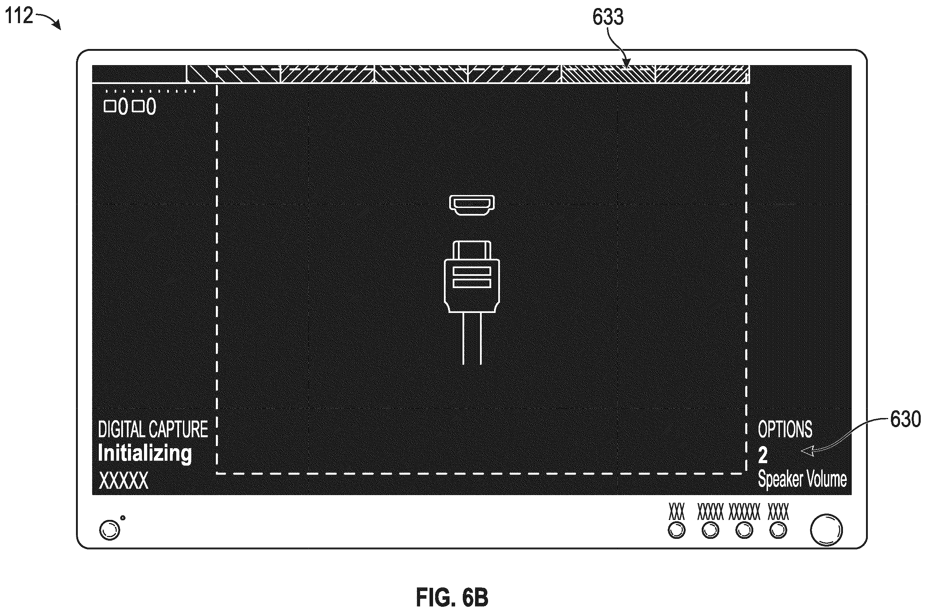

FIG. 5 illustrates examples of the different modes of a medical display device 112, according to some aspects. The medical display device 112 may change to a different display mode after static content has been displayed for a timeout period. For example, a medical display device 112 may operate in an imaging mode 502 by default until static content has been received and displayed. The static content may include image and video capture device static content 504, which may be a "no signal image" or any other image generated by the image and video capture device 108 that is found to match a pre-determined image representation. In some examples, the static content may include general static content 506, which can be any content comprising constant pixel values and/or luminance for a timeout period or for a given number of continuous frames, as described with respect to FIG. 6B.

If image and video capture device static content 504 is displayed for a timeout period T1, medical display device 112 may automatically switch to a first static imaging mode 508. In some examples, switching to first static imaging mode 508 may involve dimming the brightness of medical display device 112 and/or displaying a message, such as "Still content detected. Brightness decreased." If general static content 506 is displayed for a timeout period T2, medical display device 112 may automatically switch to the first static imaging mode 508. In some examples, the detection of general static content 506 may cause the medical display device 112 to enter a different static imaging mode than if image and video capture device static content 504 is detected.

After a static timeout period T3, the medical display device 112 may switch from static imaging mode 508 to static imaging mode 510. In static imaging mode 510, the medical display device 112 may display a new message, such as "Static content detected. Display will sleep in [T4] minutes." In some examples, the medical display device 112 may enter a low power mode and/or may further reduce the display brightness relative to static imaging mode 508. In some examples, there may not be a difference in the display parameters and/or functionality of medical display device 112 between static imaging mode 508 and static imaging mode 510 other than the different displayed messages and the different timeout periods. After a static timeout period T4, medical display device 112 may automatically enter a pixel refresh mode 512.

In some aspects, if dynamic content is detected or an action is performed during mode 508 or 510 and static timeout period T3 or T4, medical display device 112 may automatically return to imaging mode 502. In some examples, an action such as a button press, key press, or rotation of a knob may cause medical display device 112 to return to imaging mode 502. In some examples, a timer 514 may run in the background while medical display device 112 is in use. The medical display device 112 may be configured such that, after a usage period T5 has elapsed, the medical display device 112 automatically enters pixel refresh mode 512 and performs a pixel refresh regardless of the presence or absence of static content.

In some examples, the timeout periods T1 through T4 and usage period T5 may be configured and measured in units of time (*e.g*., seconds, minutes, hours, etc.), or they may be configured and measured in terms of the number of image frames. In some examples, any of the timeout periods T1-T4 may be 1-60 minutes, 60-120 minutes, 30-90 minutes, 2-4 hours, 4-6 hours, 6-8 hours, or 8-10 hours. In some examples, any of the timeout periods T1-T4 may be greater than or equal to 1 minute, 30 minutes, 45 minutes, 60 minutes, 90 minutes, 2 hours, 4, hours, 6 hours, or 8 hours. In some examples, any of the timeout periods T1-T4 may be less than or equal to 1 minute, 30 minutes, 45 minutes, 60 minutes, 90 minutes, 2 hours, 4, hours, 6 hours, or 8 hours. In some examples, usage period T5 may be 1-20 hours, 20-40 hours, 40-60 hours, 60-80 hours, or 80-100 hours. In some examples, usage period T5 may be greater than or equal to 10, 20, 30, 40, 50, 60, 70, 80, 90, or 100 hours. In some examples, usage period T5 may be less than or equal to 10, 20, 30, 40, 50, 60, 70, 80, 90, or 100 hours. In some examples, timeout periods T1-T4 and/or usage period T5 may be omitted. In other words, the medical display device 112 may be configured to stay in imaging mode 502 without automatically switching to a static imaging mode. In some examples, the medical display device 112 may be user configurable. For example, a user may toggle between enabling or disabling the automatic switching of display device 112 from imaging mode 502 to a static imaging mode via user interface 214.

In some examples, T1 and T2 may have a default duration, such as 45 minutes, T3 may have a default duration of 1 hour, T4 may have a default duration of 2 minutes, and T5 may have a default duration of 50 hours. In some examples, timeout periods T1-T4 and/or usage period T5 may be configurable by a user. For example, a user may be able to configure timeout periods T1-T4 and/or usage period T5 via user interface 214. Particularly, a user may configure T1 to be different from T2. In other words, a user may choose to have general static content 506 be displayed longer than image and video capture device static content 504 before medical display device 112 enters a static imaging mode. It may be advantageous to have general static content 506 displayed longer than image and video capture device static content 504 because the user may need to look at general static content 506 for a longer period of time, such as if general static content 506 includes a pre-operative image. In contrast, a user may not need to look at image and video capture device static content 504 for as long, such as when the displayed content includes a general "no signal" image. Also, the detection of image and video capture device static content 504 may require monitoring a fewer number of pixels, since content such as a "no signal" image will typically appear in the same region of medical display device 112 each time. Therefore, the medical display device 112 can enter a static imaging mode faster for image and video capture device static content 504. The medical operator may configure T1 and T2 to suit their viewing preferences or the needs of a given imaging session. Similarly, T3 and/or T4 may be configured to have different durations depending on the type of static content received.

Aspects of the disclosure may comprise a medical display device that includes a video processing controller. The video processing controller may be configured to perform one or more steps of method 400. For example, the video processing controller may be configured to: receive an image (step 402), generate an image representation (step 406), and compare the image representation to a pre-determined image representation (step 408). Additionally, or alternatively, in some aspects, one or more other devices (*e.g.*, an IP encoder, an image and video capture device 108, an IP decoder, a device console, a medical device) in the system may be configured to perform one or more steps of method 400.

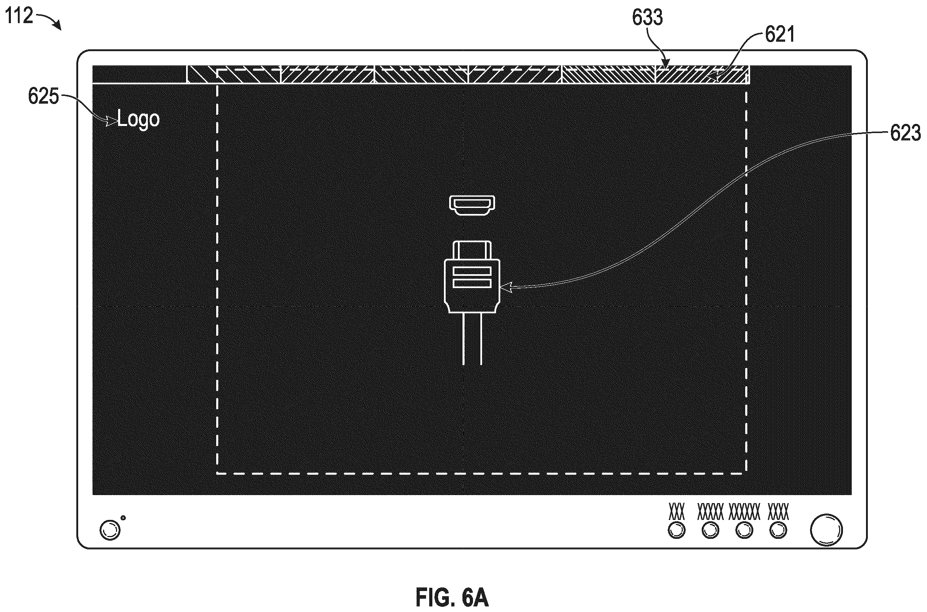

FIG. 6A illustrates an example medical display device configured to display an image comprising static content, according to some aspects. The medical display device 112 may be an OLED display, for example. The figure shows the medical display device 112 displaying an example "no signal" image. The "no signal" image may comprise one or more visuals including, but not limited to, a color bar 621, a status depiction 623, and/or a logo 625. In some examples, the color bar 621 may comprise a plurality of colors. The color bar 621 may be used for checking the image representation. The status depiction 623 may indicate a status of a system configuration. For example, in some aspects, the status depiction 623 may show a depiction of a connector, such as an HDMI cable, or a depiction of a Wi-Fi symbol that may indicate that a medical device is disconnected (*e.g*., via input port 208, wired or wirelessly) from the image and video capture device 108. Additionally, or alternatively, the status depiction 623 may show a depiction of a multimedia cable that may indicate that the image and video capture device 108 is powered off or may show a depiction of a camera that may indicate that the camera is decoupled.

**In** some examples, the image received by the medical display device 112 may comprise a region of interest 633, and the image representation may be generated based on at least the region of interest 633 of the received image. In some aspects, the region of interest 633 may be a subset of the image displayed by the medical display device 112, as shown in FIG. 6A.

**In** some instances, it may be desirable to operate the medical display device 112 in static imaging mode even if some regions of the received image comprise dynamic content. The dynamic content may comprise overlays, for example. FIG. 6B illustrates an example medical display device configured to display overlays, according to some aspects. For example, the image received by the medical display device 112 may comprise a region of interest 633 and other region 630. The other region 630 may comprise dynamic content, such as one or more overlays. Although the other region 630 may comprise dynamic content, the medical display device 112 may determine that the region of interest 633 comprises static content and may operate in static imaging mode based on the region of interest 633.

**In** some aspects, the image representation may be generated based on the region of interest 633. The parameters (*e.g*., size, location, shape, etc.) of the region of interest 633 may be pre-determined or may be dynamically configurable (*e.g*., by a medical operator, system administrator, a third-party, etc.). For example, the region of interest 633 may be located in a central region of the image, as shown in the figure. In some aspects, the region of interest 633 may be located in a non-central region of the image, such as at the bottom right corner, top left corner, etc. Examples of the disclosure may comprise the parameters of the region of interest 633 being configurable based on user input (*e.g*., manual input received by the medical display device 112, information from a user profile, etc.).

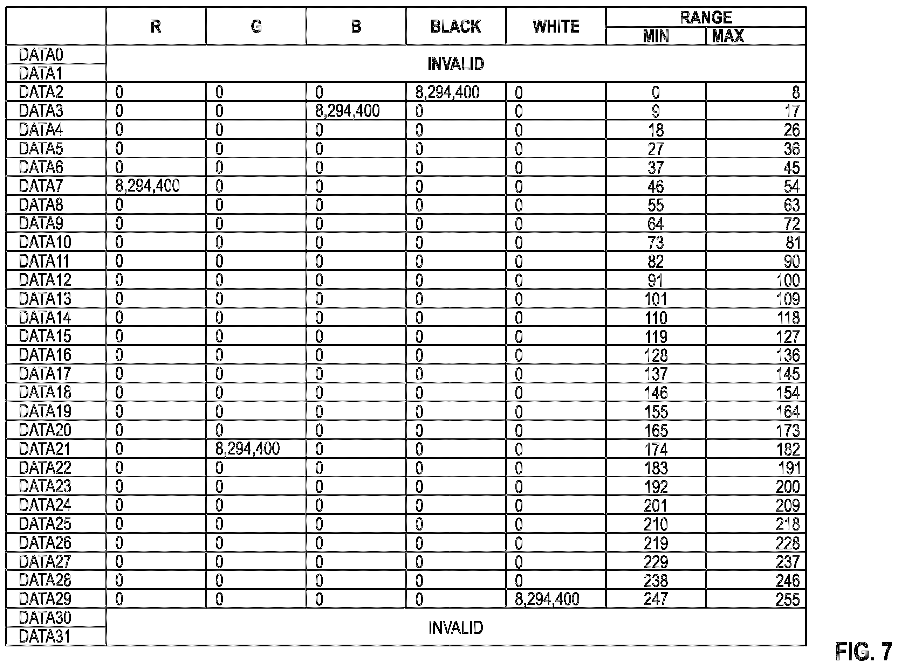

In some examples, the image representation of an image may be an image histogram. FIG. 7 illustrates an example table showing an image histogram of an image to be displayed by the medical display device, according to some aspects. The image may be divided into multiple sub-regions. For example, the table of FIG. 7 results from an image that has been divided into 32 sub-regions: Data 2, Data 3, Data 4, . . . Data 29 represent the sub-regions of the region of interest 633 (sub-regions of interest) and Data 0, Data 1, Data 30, and Data 31 ("Invalid") represent the sub-regions of the other region 630. The pixels of the sub-regions of interest are indicated in the Range columns. And each sub-region of interest comprises a plurality of pixels, such as red pixels (R), green pixels (G), blue pixels (B). The numbers in the R, G, and B columns represent the number of red pixels, green pixels, and blue pixels, respectively, in a sub-region of interest. In some aspects, luminance values may be determined for each sub-region of interest, as shown by the values of the Black and White columns of the table. As a non-limiting example, a luminance value for a given sub-region of interest may be determined by multiplying the number of red pixels by a (red) multiplier, multiplying the number of green pixels by a (green) multiplier, multiplying the number of blue pixels by a (blue) multiplier, and then summing the multiplied values to obtain the luminance value for the sub-region of interest.

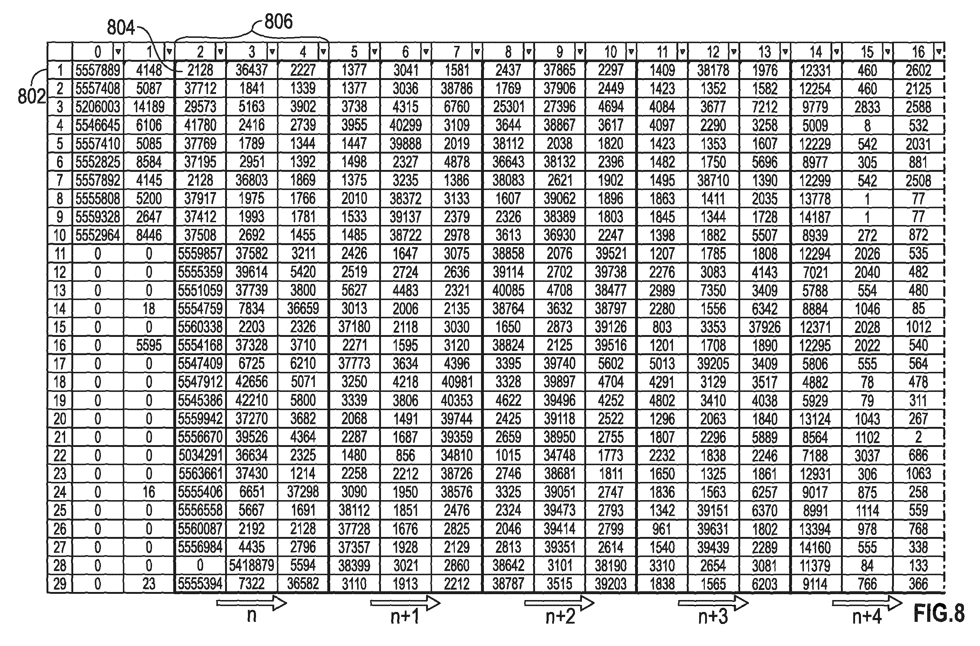

FIG. 8 illustrates another example table showing an image histogram, according to some aspects of the disclosure. Like the example of FIG. 7, the image histogram represents an image that has been divided into 29 sub-regions 802. For each sub-region 802, a plurality of data items 804 can be compared over an interval 806 (*e.g*., every second, every frame). The plurality of data items may be luminance values , obtained from pixel values as in FIG. 7. If a given data item continuously repeats over a threshold number of intervals 806 (for example, three times for five intervals), the image may be considered to include static content. Otherwise, the image may be considered to include dynamic content. The interval size and/or threshold number of repeats for an image to be judged as including static content may be preconfigured as part of the configuration data (*e.g*., firmware) for image and video capture device 108.

As discussed above, comparing the image representation to the pre-determined image representation may comprise comparing values of sub-regions of interest in the image representation to values of corresponding sub-regions of interest in the pre-determined image representation. In some aspects, the properties (*e.g*., number of sub-regions of interest, size of the sub-regions of interest, number of pixels in each sub-region of interest, size/shape of the region of interest, etc.) may be the same for the image representation and the pre-determined image representation. In some aspects, the image representation comprises an image histogram, and the pre-determined image representation comprises a pre-determined image histogram.

In some aspects, the image representation and the pre-determined image representation may be divided into sub-regions according to the properties (*e.g*., resolution) of the image to be displayed by the medical display device 112. For example, the image representation and pre-determined image representation may be divided into a greater number of sub-regions when the image resolution is high (*e.g.*, 4K), and fewer sub-regions when the image resolution is low (*e.g.*, 720p). In some aspects, since the images to be the displayed by the medical display device 112 may have different resolutions, the pre-determined image representation may correspond to an average of pre-determined image representations having a plurality of resolutions. In this manner, a pre-determined image representation may be used for comparison to the image representation independent of the resolution of the image to be displayed.

In some aspects, examples of the disclosure comprise a frame comparison-based method that compares a plurality of video frames (images) to determine that the image to be displayed comprises static content. The frame comparison-based method may comprise generating image representations for regions of interests for a plurality of images, where the plurality of images are from different video frames. The method may compare the image representations to each other to determine that they correspond (*e.g*., have values that match, or are within a threshold deviation) to each other. For example, the method may determine that the image comprises static content when a first image representation of a first video frame corresponds to a second image representation of a second video frame. In some aspects, the plurality of images may be received within a certain time period. For example, the first video frame and the second video frame may be consecutive video frames. Examples of the disclosure include comparing the image representation(s) of one or more video frames to a pre-determined image representation.

Examples of the disclosure also comprise a bypass signal-based method, where the medical display device 112 receives one or more bypass signals. The bypass signal may indicate that the image received comprises static content. In some aspects, when the medical display device 112 receives a bypass signal, the medical display device 112 may be configured to forego one or more steps of the disclosed method. In some aspects, the bypass signal may be a static content bypass signal that causes the medical display device 112 to forego comparing the image representation to a pre-determined image representation, and forego determining that the image representation corresponds to the pre-determined image representation. The static content bypass signal may be a direct indication to the medical display device 112 that the image received comprises static content, and as a result, the medical display device 112 may bypass the steps for determining that the image received comprises static content.

In some aspects, the bypass signal may be an imaging mode bypass signal that causes the medical display device 112 to operate in imaging mode. In some instances, the image received by the medical display device 112 may comprise static content, but the medical operator may not want the medical display device 112 to operate in static imaging mode. For example, the medical operator (*e.g*., surgeon) may use the medical display device 112 to view a still image (*e.g.*, an x-ray image). The imaging mode bypass signal may allow the medical operator to view the image comprising static content without having the medical display device 112 display messages (or use an indicator such as an LED indicator), operate in a low power mode (*e.g.*, cause a reduced brightness of the medical display device), and/or perform a pixel refresh. The imaging mode bypass signal may be direct indication to the medical display device 112 that it should operate in imaging mode even though the image received comprises static content. In some instances, in receiving the imaging mode bypass signal, the medical display device 112 may bypass the steps for determining that the image received comprises static content.

As discussed above, the medical display device 112 may operate in static imaging mode, taking one or more steps to minimize exposure of the medical display device 112 to static content. For example, in static imaging mode, the medical display device 112 may operate in a low power mode (*e.g.*, cause a reduced brightness of the medical display device) and/or perform a pixel refresh with the medical display device powered off. In some aspects, the medical display device 112 may display a message indicating that the medical display device 112 is still operating, but has been temporarily in static imaging mode to preserve its integrity.

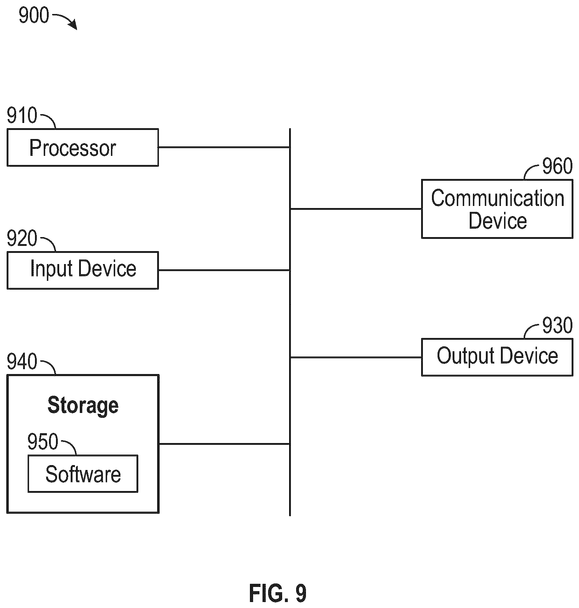

FIG. 9 illustrates an example computing system, in accordance with some examples, that can be used for performing any of the methods described herein, including method 400 of FIG. 4, and can be used for any of the systems described herein, including the medical display device 112, the image and video capture device 108, the device console 344, and/or a medical device 302. System 900 can be a computer coupled to a network, which can be, for example, an operating room network or a hospital network. System 900 can be a client computer or a server. As shown in FIG. 9, system 900 can be any suitable type of controller (including a microcontroller) or processor (including a microprocessor) based system, such as an embedded control system, personal computer, workstation, server, or handheld computing device (portable electronic device) such as a phone or tablet. The system can include, for example, one or more of processor 910, input device 920, output device 930, storage 940, or communication device 960.

Input device 920 can be any suitable device that provides input, such as a touch screen, keyboard or keypad, mouse, gesture recognition component of a virtual/augmented reality system, or voice-recognition device. Output device 730 can be or include any suitable device that provides output, such as a touch screen, haptics device, virtual/augmented reality display, or speaker.

Storage 940 can be any suitable device that provides storage, such as an electrical, magnetic, or optical memory including a RAM, cache, hard drive, removable storage disk, or other non-transitory computer readable medium. Communication device 960 can include any suitable device capable of transmitting and receiving signals over a network, such as a network interface chip or device. The components of the computer can be coupled in any suitable manner, such as via a physical bus or wirelessly.

Software 950, which can be stored in storage 940 and executed by processor 910, can include, for example, the programming that embodies the functionality of the present disclosure (*e.g.*, as embodied in the devices as described above). For example, software 950 can include one or more programs for performing one or more of the steps of the methods disclosed herein.

Software 950 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 940, that can contain or store programming for use by or in connection with an instruction execution system, apparatus, or device.

Software 950 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic, or infrared wired or wireless propagation medium.

System 900 may be coupled to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 900 can implement any operating system suitable for operating on the network. Software 950 can be written in any suitable programming language, such as C, C++, C#, Java, or Python. In various examples, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for operating a medical display device, the method comprising:
receiving an image for display by the medical display device;
generating an image representation of at least a region of interest of the received image;
comparing the image representation to a pre-determined image representation;
determining that the image representation corresponds to the pre-determined image representation; and
operating the medical display device in a static imaging mode.

2. The method of claim 1, wherein the region of interest of the received image is a subset of the received image, and the image optionally comprises the region of interest and an other region, wherein the other region comprises dynamic content.

3. The method of claim 1 or 2, wherein the region of interest of the received image comprises one or more of: a color bar, a status depiction, a logo, a depiction of a connector, a depiction of a Wi-Fi symbol, a depiction of a multimedia cable, and a depiction of a medical device.

4. The method of claim 1, 2 or 3, further comprising:
dynamically configuring parameters of the region of interest of the received image.

5. The method of any of claims 1-4, further comprising:
generating the pre-determined image representation by averaging a plurality of pre-determined image representations having a plurality of resolutions.

6. The method of any of claims 1-5, wherein generating the image representation comprises determining luminance values for the region of interest of the received image.

7. The method of any of claims 1-6, wherein the pre-determined image representation corresponds to a third-party pre-determined image representation.

8. The method of any of claims 1-7, wherein the image representation comprises an image histogram and the pre-determined image representation comprises a pre-determined image histogram.

9. The method of any of claims 1-8, wherein operating the medical display device in the static imaging mode comprises one or more of:
causing the medical display device to display a message;
operating the medical display device in a low power mode; and
performing a pixel refresh with the medical display device powered off.

10. The method of any of claims 1-9, further comprising:
waiting a timeout period after determining that the image representation corresponds to the pre-determined image representation before operating the medical display device in the static imaging mode.

11. The method of any of claims 1-10, further comprising:
receiving a bypass signal; and
foregoing the comparing step and the determining step.

12. A system for displaying a medical image or video, the system comprising:
a medical display device configured to:
receive an image for display;
generate an image representation of at least a region of interest of the received image;
compare the image representation to a pre-determined image representation;
determine that the image representation corresponds to the pre-determined image representation; and
operate in a static imaging mode.

13. The system of claim 12, wherein the image comprises the region of interest and another region, and wherein the region of interest is a subset of the received image, and the other region comprises dynamic content.

14. The system of claim 12 or 13 the system further comprising:
an image and video capture device;
a device console; and
a medical device,
wherein the region of interest of the received image comprises static content when the image and video capture device is decoupled from the device console, or the device console is decoupled from the medical device.

15. A computer program product including computer-implementable instructions configured to be executed by one or more processors of a system, wherein executing the instructions causes the system to:
receive an image for display by a medical display device;
generate an image representation of at least a region of interest of the received image;
compare the image representation to a pre-determined image representation;
determine that the image representation corresponds to the pre-determined image representation; and
operate the medical display device in a static imaging mode.
